Europäisches Patentamt

European Patent Office

Office européen des brevets

(11) Publication number: **0 143 478**
**B1**

## (12) EUROPEAN PATENT SPECIFICATION

(45) Date of publication of patent specification: **22.07.87**

(21) Application number: **84201425.0**

(22) Date of filing: **04.10.84**

(51) Int. Cl.⁴: **A 61 K 33/24**

(54) Stable, aqueous, acidic solution of cis-platinum, suitable for injection.

(30) Priority: **24.10.83 NL 8303657**

(43) Date of publication of application:
**05.06.85 Bulletin 85/23**

(45) Publication of the grant of the patent:
**22.07.87 Bulletin 87/30**

(84) Designated Contracting States:
**AT BE CH DE FR GB IT LI LU NL SE**

(56) References cited:
GB-A-2 021 946
GB-A-2 074 028
US-A-4 310 515

CHEMICAL ABSTRACTS, vol. 91, no. 6, August
6, 1979, page 316, column 1, abstract no.
44464n, COLUMBUS, OHIO (US). - A.A. HINCAL
et al.: "cis-Platin stability in aqueous parenteral
vehicles"

The file contains technical information
submitted after the application was filed and
not included in this specification

(73) Proprietor: **Pharmachemie B.V.**
**Waarderweg 78**
**Haarlem (NL)**

(72) Inventor: **Nijkerk, Alfred James**
**Tigris 2**
**Amstelveen (NL)**

(74) Representative: **Elzas, Salomon, Drs. et al**
**Octrooibureau Polak & Charlouis Laan Copes**
**van Cattenburch 80**
**NL-2585 GD The Hague (NL)**

(56) References cited:
CHEMICAL ABSTRACTS, vol. 95, no. 23,
December 7, 1981, page 25, column 1, abstract
no. 197173z, COLUMBUS OHIO (US).- C.L.
LITTERST: "Alternations in the toxity of cis-
dichlorodiammine-platinum-II and in tissue
localization of platinum as a function of
sodium chloride concentration in the vehicle of
administration" on"

Courier Press, Leamington Spa, England.

## Description

This invention relates to a stable, aqueous, hydrochloric acid-containing solution of cis-platinum (chemical name cis-dichlorodiammine platinum), suitable for injection, as well as to a process for preparation thereof.

Such a solution is known from Dutch patent application 7 901 283 (GB—A—2021946). According to this patent application the pH of the solution is adjusted to a value in the range of 2.0—3.0, preferably 2.3—2.7. Although the protection claimed in said patent application have not been restricted thereto, it appears from the specific examples that in such a solution also mannitol is incorporated. This also is logical, for the process disclosed there constitutes an improvement over the form of the preparation which was usual before that time, wherein the cis-platinum was delivered as a white lyophilized powder in combination with sodium chloride, mannitol and hydrochloric acid, which powder had to be reconstituted with sterile water. Moreover, these combinations had to be stored under cooling, and the reconstitution of course was a tedious procedure, and the reconstituted compositions had a useful life of only about 20 hours at 22°C. It is for this reason that according to Dutch patent application 7901283 a stable, sterile aqueous solution of cis-platinum is provided with the abovementioned low pH, because according to that patent application the composition only then shows a sufficient stability. Now, the side effects of anti-tumour agents are already quite serious and a pH in such a strongly acidic range of course does not make the injection more agreeable.

Moreover, the teachings of Dutch patent application 7 901 283 are in agreement with an article in Journal of the Parenteral Drug Association, May/June 1979, pages 107—115. This article discusses the stability of cis-platinum in aqueous media suitable for parenteral administration. It appears from this article that the stability of cis-platinum therein usually is measured in hours and that the decomposition of the cis-platinum is promoted by a pH higher than 7 (see page 113), whereas solutions of common salt, dextrose and mannitol, for instance, usually show pH values in the range of 4—6 as a result of absorbed carbon dioxide and the like, so that they do not show an additional acceleration of the decomposition. However, it appears that a pH of 4 for such a neutral solution would be an extremely low value which normally will not be attained, and it does not appear from said article that experiments have been carried out at such a low pH.

U.S.—A—4 310 515 which is a C.I.P. of the U.S. application corresponding to Dutch patent application 7 901 283 additionally discloses in the experimental part thereof comparative experiments with mannitol-containing solutions of varying pH values, from which it appears that such a cis-platinum solution having a pH of 3.5 is insufficiently stable. Additionally, this U.S. patent discloses experiments with cis-platinum solutions not containing mannitol, wherein, however, only solutions of pH 2.3 and 2.4 were tested. Accordingly, the understanding that without mannitol solutions of higher pH may be used fails in this U.S. patent.

A proposal of different nature is found in Dutch patent application 8 101 531 (G.B.—A 2 074 028). According to that patent application a mixture of 30—95% of polyethylene glycol or methoxy polyethylene glycol and 5—70% of water is used as the solvent, and the solution also contains a source of chloride ions. Preferably, the solvent is a mixture of about 80—95% polyethylene glycol or methoxy polyethylene glycol and about 5—20% water. As appears from the specification and examples, the composition should not necessarily contain HCl, although this is the case in many of the examples. No mention is made of a pH value and of course a pH value is not so significant in a predominantly non-aqueous composition. The said proposal indeed leads to a stable concentrated solution, but the drawback is the presence of the polyethylene glycol (or methoxy polyethylene glycol). In general physicians prefer that the cis-platinum composition to be administered contains as little foreign substances as possible. In view of the strong side effects of this anti-cancer agent which differs from patient to patient, an individual dosage of adjuvants is to be highly preferred and for this reason these adjuvants are preferably administered separately. Moreover, the treatment with substances which should reduce the noxious side effects of the cis-platinum, should often be carried out on a trial and error base.

Consequently, the problem still exists to prepare a stable solution of cis-platinum which contains as few other substances as possible, and is not too extremely acidic.

Surprisingly, it has now been found that this object can be attained with the aid of a solution which only contains cis-platinum, common salt and hydrochloric acid.

To this end the invention provides a stable, aqueous, hydrochloric acid-containing solution of cis-platinum, suitable for injection, which is characterized in that this solution contains no more than 1.0 mg/ml cis-platinum and sodium chloride in an amount of 0.4—1%, has a pH of 3.2—5.0 and is substantially free of other ingredients.

With "substantially free of other ingredients" is meant here that the solution preferably does not contain any further ingredients, but that, if necessary, small amounts of adjuvants, as are perhaps desired by individual physicians, can be added, provided that the amount is not greater than that of the cis-platinum in the solution.

Preferably, the pH of the solution is 3.2—3.5. Furthermore, it is preferred that the sodium chloride concentration of the solution closely corresponds to that of a physiological salt solution.

The solution of this invention can be prepared in any desired way. A practical method is to dissolve the

2

cis-platinum in desired concentration in a solution of the sodium chloride in a part of the water, whereafter the pH of this solution is brought to the desired value by means of hydrochloric acid, and then the solution is diluted with water to the desired concentration.

If the cis-platinum content is higher than 0.6 mg/ml, the cis-platinum may cristallize at temperatures below 20°C. Therefore, it is preferred that the cis-platinum content is no more than 0.6 mg/ml and even more particularly not more than 0.5 mg/ml. However, if temperatures below about 20°C are not to be expected during shipping and storage of the solution, contents up to the usual 1.0 mg/ml can also be used.

An example of the preparation is as follows:

450 g of sodium chloride are dissolved in 25 l of distilled and pyrogene-free water. Then 25 g of cis-platinum (content 100%) are dissolved in the so obtained solution. Thereafter 60 ml of 10% hydrochloric acid are added, whereby the pH is adjusted to a value of 3.2—3.5. Subsequently, distilled and pyrogene-free water is added to the solution to a volume of 50 l. Finally, the entire mixture is filtered aseptically through a sterile membrane filter of 0.22 um into sterile containers.

The so obtained sterile solution can be introduced in the usual way into sterile vials or ampoules for injection. These vials or ampoules can contain for instance 10, 25 or 50 mg of the active compound.

It is still remarked that, although as mentioned hereinabove, further small amounts of additives in principle may be present, which perhaps will be the case in future, if a certain trend is formed among a group of physicians, it is now preferred that the composition does not contain any further ingredients.

The stability of the so obtained solutions was tested by HPLC in an analogous way to that described on pages 108 and 109 of the article in Journal of the Parenteral Drug Association. During the analytical research in preparation thereto, it was found however that it was not necessary to use both a cation-exchange and an anion-exchange column, but that the use of the anion-exchange column Whatman Partisil-10 SAX mentioned in the article, is sufficient. This was established by preparing a number of test solutions and examining these according to both methods. It appeared that the determination of the cis-platinum content with the simpler method yielded results which were completely comparable with the results of the method with two columns. The method with one column not only has the advantage that it is simpler, but also that the regeneration is much simpler. As the mobile phase the same mixture was chosen as in the article, 30% acetic acid/sodium acetate-buffer (pH 5.6, 0.01 M) and 70% methanol. The elution rate was 1.2 ml per minute and the injected volume was 100 µl. The detection was carried out at 303 nm. The calculation was carried out with the aid of a Spectrum-Physics integrator. In this chromatography the composition products and trans-platinum are separated from the cis-platinum and eluted with the solvent front.

In this way the decomposition of the cis-platinum was followed at various temperatures and various pH values. As typical, the results are given here which were obtained at pH 4.0. The tests were carried out with dosages of 10 ml and of 50 ml. During all the tests, the solutions remained clear and they had a colour value of <G6.

| | Time, weeks | 10 ml | | 50 ml | |
|---|---|---|---|---|---|
| | | pH | Content, % | pH | Content, % |
| Room temperature: | 0 | 4.0 | 101.7 | 4.0 | 101.7 |
| | 4 | 4.0 | 101.2 | | |
| | 21 | 4.1 | 100.0 | 4.1 | 100.1 |
| | 29 | 4.0 | 99.9 | 4.0 | 99.6 |
| 40°C | 3 | 4.1 | 99.9 | | |
| | 12 | 4.4 | 98.4 | | |
| 50°C | 4 | 4.6 | 99.6 | | |

**Claims for the Contracting States: BE CH DE FR GB IT LI LU NL SE**

1. Stable, aqueous, hydrochloric acid-containing solution of cis-platinum, suitable for injection, characterized in that the solution contains no more than 1.0 mg/ml of cis-platinum and sodium chloride in an amount of 0.4—1%, has a pH of 3.2—5.0 and is substantially free from other ingredients.

2. Solution according to claim 1, characterized in that the solution has a pH of 3.2—3.5.

3. Solution according to claim 1 or 2, characterized in that the sodium chloride concentration substantially corresponds to that of a physiological salt solution.

4. A process for preparing a solution according to claims 1—3, characterized in that the cis-platinum is dissolved in a solution of the sodium chloride in a part of the water, the pH of this solution is adjusted to the desired value by means of hydrochloric acid, and the solution is then diluted with water to the desired concentration.

**Claims for the Contracting State: AT**

1. Process for preparing a stable, aqueous, hydrochloric acid-containing solution of cis-platinum suitable for injection, characterized by incorporating in said solution no more than 1.0 mg/ml of cis-platinum and sodium chloride in an amount of 0.4—1%, adjusting the pH at 3.2—5.0 and keeping the solution substantially free of other ingredients.

2. A process according to claim 1, characterized by adjusting the solution at a pH of 3.2—3.5.

3. A process according to claim 1 or 2, characterized by adding sodium chloride in a concentration which substantially corresponds to that of a physiological salt solution.

4. A process according to claims 1—3, characterized by dissolving the cis-platinum in a solution of the sodium chloride in a part of the water, adjusting the pH of this solution to the desired value by means of hydrochloric acid, and then diluting the solution with water to the desired concentration.

**Patentansprüche für die Vertragsstaten: BE CH DE FR GB IT LI LU NL SE**

1. Stabile, wässrige, Chlorwasserstoff enthaltende Lösung von Cisplatin, welche für Injection geeignet ist, dadurch gekennzeichnet, dass die Lösung nicht mehr als 1,0 mg/ml Cisplatin und Natriumchlorid in einer Menge von 0,4—1% enthält, einen pH-Wert von 3,2—5,0 aufweist und praktisch frei von anderen Bestandteilen ist.

2. Lösung nach Anspruch 1, dadurch gekennzeichnet, dass die Lösung einen pH-Wert von 3,2—3,5 aufweist.

3. Lösung nach Anspruch 1 oder 2, dadurch gekennzeichnet, dass die Natriumchloridkonzentration praktisch derjenigen einer physiologischen Salzlösung entspricht.

4. Verfahren zur Herstellung einer Lösung gemäss den Ansprüchen 1—3, dadurch gekennzeichnet, dass das Cisplatin aufgelöst wird in einer Lösung des Natriumchlorids in einem Teil des Wassers, der pH-Wert dieser Lösung mittels Chlorwasserstoffsäure auf dem gewünschten Wert eingestellt wird, und die Lösung dann mit Wasser zu der gewünschten Konzentration verdünnt wird.

**Patentansprüche für den Vertragsstaat: AT**

1. Verfahren zur Herstellung einer stabilen, wässrigen, Chlorwasserstoffsäure enthaltende Lösung von Cisplatin, welche sich für Injektion eignet, dadurch gekennzeichnet, dass man in diese Lösung nicht mehr als 1,0 mg/ml Cisplatin und Natriumchlorid in einer Menge von 0,4—1% aufnimmt, den pH-Wert auf 3,2—5,0 einstellt und die Lösung praktisch frei von anderen Bestandteilen hält.

2. Verfahren nach Anspruch 1, dadurch gekennzeichnet, dass man die Lösung auf einen pH-Wert von 3,2—3,5 einstellt.

3. Verfahren nach Anspruch 1 oder 2, dadurch gekennzeichnet, dass man Natriumchlorid in einer Konzentration zusetzt, welche derjenigen einer physiologischen Salzlösung praktisch entspricht.

4. Verfahren nach Ansprüchen 1—3, dadurch gekennzeichnet, dass man das Cisplatin in einer Lösung des Natriumchlorids in einem Teil des Wassers auflöst, den pH-Wert dieser Lösung mittels Chlorwasserstoffsäure auf den gewünschten Wert einstellt und die Lösung dann mit Wasser zu der gewünschten Konzentration verdünnt.

**Revendications pour les états contractants: BE CH DE FR GB IT LI LU NL SE**

1. Solution stable, aqueuse de cis-platine contenant de l'acide hydrochlorique et appropriée à l'injection, caractérisé en ce que la solution contient 1,0; mg/ml de cis-platine au maximum et le chlorure de sodium en quantité de 0,4—1%, possède un pH de 3,2—5,0 et est pratiquement libre d'autres ingrédients.

2. Solution selon la revendication 1, caractérisé en ce que la solution possède un pH de 3,2—3,5.

3. Solution selon la revendication 1 ou 2, caractérisé en ce que la concentration du chlorure de sodium correspond pratiquement à celle dans un sérum physiologique.

4. Procédé pour la préparation d'une solution selon les revendications 1—3, caractérisé en ce que le cis-platine est dissous dans une solution du chlorure de sodium dans une partie de l'eau, pe pH de cette solution est apporté à la valeur désirée à l'aide de l'acide hydrochlorique, et la solution est puis diluée avec de l'eau jusqu'à la concentration desirée.

**0 143 478**

1. Procédé de préparation d'une solution stable, aqueuse de cis-platine contenant de l'acide hydrochlorique et appropriée à i'injection, caractérisé en ce qu'on incorpore dans cette solution 1,0 mg/ml de cis-platine au maximum et du chlorure de sodium en quantité de 0,4—1%, qu'on porte le pH à 3,2—5,0 et maintient la solution pratiquement libre d'autres ingrédients.

2. Procédé selon la revendication 1, caractérisé en ce qu'on porte la solution à une pH de 3,2—3,5.

3. Procédé selon la revendication 1 ou 2, caractérisé en ce qu'on ajoute le chlorure de sodium dans une concentration qui correspond pratiquement à celle d'un sérum physiologique.

4. Procédé selon les revendications 1—3, caractérisé en ce qu'on dissout le cis-platine dans une solution du chlorure de sodium dans une partie de l'eau, porte le pH de cette solution à la valeur désirée à l'aide de l'acide hydrochlorique et puis dilue la solution avec l'eau jusqu'à la concentration désirée.